(19)

Europäisches Patentamt

European Patent Office

Office européen des brevets

(11) **EP 4 176 809 A1**

(12) **EUROPEAN PATENT APPLICATION**

(43) Date of publication:
**10.05.2023 Bulletin 2023/19**

(21) Application number: **21206924.9**

(22) Date of filing: **08.11.2021**

(51) International Patent Classification (IPC):
**A61B 5/11** (2006.01)    **A61B 5/00** (2006.01)
**A61G 7/018** (2006.01)    **G16H 40/00** (2018.01)

(52) Cooperative Patent Classification (CPC):
**A61B 5/1113; A61B 5/1128; A61B 5/6889; A61B 5/7221; G16H 30/20; G16H 30/40; G16H 40/63**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **VAN DER HEIDE, Esther Marjan**
  **Eindhoven (NL)**

• **ZUO, Fei**
  **Eindhoven (NL)**
• **BOUTS, Mark Jacobus Rosalie Joseph**
  **Eindhoven (NL)**
• **BRESCH, Erik**
  **Eindhoven (NL)**

(74) Representative: **Philips Intellectual Property & Standards**
**High Tech Campus 52**
**5656 AG Eindhoven (NL)**

(54) **DEVICE, SYSTEM AND METHOD FOR MONITORING A SUBJECT**

(57)    The present invention relates to a device, system and method for monitoring a subject. The device comprises a processing unit that is configured to detect, within the obtained image data, one or more body parts of the subject, whose image data allow determination of a desired measurement parameter, determine a level of occlusion of the detected one or more body parts, determine the desired measurement parameter from the image data of at least one of the detected one or more body parts, and compute reliability information of the determined measurement parameter based on the detected level of occlusion of the at least one of the detected one or more body parts whose image data have been used to determine the measurement parameter.

EP 4 176 809 A1

100

101 — obtain image data

102 — detect body part(s)

103 — determine level of occlusion

104 — determine measurement parameter

105 — compute reliability information

106 — output measurement parameter and reliability information

FIG.3

**Description**

FIELD OF THE INVENTION

The present invention relates to a device, system and method for monitoring a subject, such as a patient, an elderly person in a rest home, a child, a premature baby etc.

BACKGROUND OF THE INVENTION

Camera-based monitoring is an unobtrusive manner to monitor patients and is increasingly being used in different care settings such as the intensive care unit (ICU). Now in the time of COVID-19 the use of a camera system can be of interest as clinical staff can keep an eye on the patient without the need to enter the room and work in protective clothing. The camera can be used to measure vital signs (e.g. respiratory rate, heart rate, Sp02, etc.), delirious movements, bed exit, pressure ulcer, facial expressions, etc. A good view on the patient without any objects or persons obscuring the image is key for reliable measurements.

The ICU room is a dynamic place with a lot of persons walking in and out of the room, with persons taking care of the patient and in close vicinity of the patient. Next to persons also medical equipment is around or even at the patient bed next to that there can be trays, television screens obscuring the view of the camera on the patient. Occlusion of the patient will restrict the reliability of camera-based measurement e.g. respiratory rate or delirium score. Knowing the occluded area and more precisely the patient body part/region can give information about the reliability of a measurement.

SUMMARY OF THE INVENTION

It is an object of the present invention to provide device, system and method for monitoring a subject that are able to determine the occluded area and the patient body part/region and to obtain information about the reliability of a measurement.

In a first aspect of the present invention a device for monitoring a subject is presented comprising:

an input interface configured to obtain image data acquired over time by an imaging unit from a field of view containing at least part of a subject;
a processing unit configured to

- detect, within the obtained image data, one or more body parts of the subject, whose image data allow determination of a desired measurement parameter,
- determine a level of occlusion of the detected one or more body parts,
- determine the desired measurement parameter from the image data of at least one of the detected one or more body parts, and
- compute reliability information of the determined measurement parameter based on the detected level of occlusion of the at least one of the detected one or more body parts whose image data have been used to determine the measurement parameter; and

an output interface configured to output the measurement parameter and the computed reliability information.

In a further aspect of the present invention a system for monitoring a subject is presented comprising:

- an imaging unit configured to acquire image data over time from a field of view containing at least part of a subject, and
- a device as disclosed herein for monitoring a subject based on the acquired image data.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

Video monitoring is increasingly gaining interest to use in a clinical setting such as the ICU. A problem is that in the room other persons such as clinical stakeholders, family members, or (medical) equipment can obscure the view on the patient. Even with a top view camera that has an optimal view on the patient, there are moments that the view is obscured. This limited view on the area/body parts of interest can result in a decrease in the reliability of the measurement of interest. The present invention enables to understand the occluded area or, more precisely, body parts, which can give information about the reliability of the measurement and can give insight in the reliability of a measurement and use of that measurement. A lower reliability score indicates that the outputted measurement parameter is less trustworthy.

In an embodiment the processing unit is configured to select the at least one of the detected one or more body parts whose image data shall be used to determine the measurement parameter based on the level of occlusion of the one or more body parts. This improves the quality and reliability of the measurement.

In another embodiment the processing unit is configured to detect a subject area in which the subject is located, in particular an area in which a bed and/or chair is located, and to detect the one or more body parts of the subject within the detected subject area. This improves the detection of the subject area within which a body part can then be detected.

Further, the processing unit may be configured to detect occluding objects and/or persons occluding at least part of the subject, bed and/or chair and to detect the subject area and/or the one or more body parts of the subject using the detected occluding objects and/or persons. For instance, the processing unit may be configured to determine, for the detected occluding objects and/or persons, a level of obscurity indicating to which extent a respective occluding object and/or person occludes or obstructs the subject area, and to use the determined level of obscurity in the computation of the reliability information of the determined measurement parameter. This makes the determination of the reliability information more precise.

The processing unit may further be configured to detect occluding objects and/or persons occluding at least part of the subject, bed and/or chair by tracking motion of objects and/or persons within the image data and determine if a tracked object and/or person is or will be an occluding object or person based on the tracked motion. This information can then be used to select a body part for making a measurement and/or to compute the reliability information.

In another embodiment the processing unit is configured to

- detect position and/or orientation of the subject,
- determine, based on the detected position and/or orientation, a weighting factor indicating how well the one or more body parts of the subject can be detected and/or how well the desired measurement parameter can be determined from the image data of at least one of the detected one or more body parts, and
- use the determined weighting factor in the computation of the reliability information of the determined measurement parameter.

This operation improves the accuracy of the computed reliability information.

In another embodiment the processing unit is configured to detect scene changes, in particular body motions and/or position changes of the subject and/or changes in occlusion of one or more body parts of the subject, and to carry out body part detection and occlusion detection only when a scene change happens. This saves computation time and effort.

The determined level of occlusion of the detected one or more body parts may include one or more of:

- identification of occluded body parts,
- number of occluded body parts,
- percentage of the occlusion of the respective body parts,
- duration of occlusion of the respective body parts,
- frequency of occlusion,
- change in overall body position of the subject, and
- type of occluding object and/or person.

The desired measurement parameter may include one or more of respiration rate, heart rate, Sp02, bed fall likelihood, bed exit detection, pressure ulcers, delirious movements, pain, facial expressions, subject motion, subject activity and blood pressure.

Different factors may be exploited in the determination of the reliability information. In an embodiment the processing unit may be configured to compute the reliability information of the determined measurement parameter based on the detected level of occlusion by reducing the reliability with increasing level of occlusion. Accordingly, the reliability is increased with reducing level of occlusion. In another embodiment the processing unit is configured to compute the reliability information of the determined measurement parameter based on the duration of occlusion in addition to the detected level of occlusion by reducing the reliability with increasing duration of occlusion.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention.
Fig. 2 shows a schematic diagram of an embodiment of a device according to the present invention.
Fig. 3 shows a schematic diagram of an embodiment of a method according to the present invention.
Fig. 4 shows a schematic diagram of another embodiment of a device according to the present invention.

DETAILED DESCRIPTION OF EMBODIMENTS

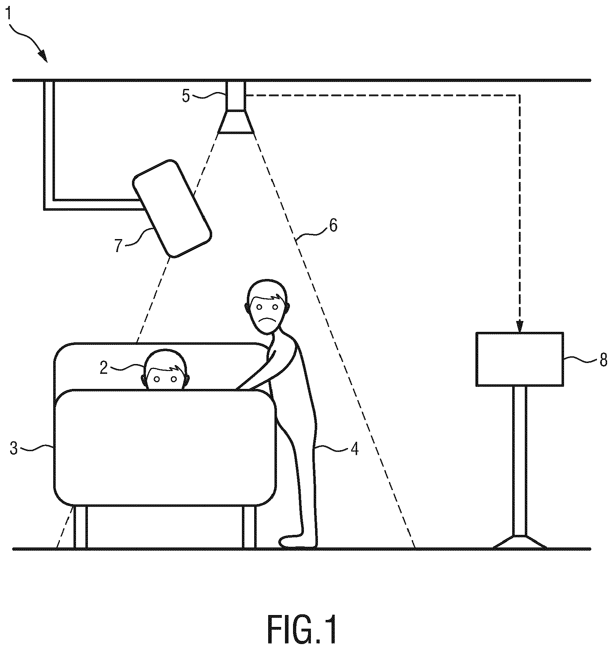

Fig. 1 shows a schematic diagram of an embodiment of a system 1 for monitoring a subject according to the present invention. In this scenario the subject 2 is a patient, e.g. in a hospital or a rest home, lying in a bed 3. Another person 4, e.g. a nurse or a visitor, is standing by the bed 3. The system 1 comprises an imaging unit 5, such as a conventional optical video camera mounted at the ceiling above the bed 3, which is configured to acquire image data over time from a field of view 6 containing at least part of the patient 2. The acquired image may simply be used for display on a monitor 7, e.g. in a central monitoring room, to monitor the patient 2, and/or may be processed by a device 8, e.g. a computer, for monitoring the subject based on the acquired image data, in particular to obtain a monitoring signal providing a particular information regarding the patient 2, e.g. if the patient 2 is still in the bed 3, the respiration rate or pulse rate of the patient 2, any activity of the patient 2, etc.

As shown in the scenario depicted in Fig. 1, the view of the imaging unit 5 can be hampered by several factors, such as an improper position of the patient 2 and/or the bed 3 in relation to the field of view 6 of the imaging unit 5, occlusions caused by objects 7 (such as a monitor or movable table) in between imaging unit 5 and patient 2 and occlusions caused by other people 4 (e.g. medical staff) in between imaging unit 5 and patient 2.

Known methods to address these challenges include visually checking the imaging unit's output and manually taking corrective action (e.g. manually changing the position or field of view of the imaging unit), which however has privacy concerns and requires skill and effort. Another known method is to provide specific workflow instructions on where to place the bed, objects, etc., which are however prone to human error and not very flexible. Further, redundancy may be provided through multiple imaging units involving however additional costs and increased system complexity.

According to the present invention, the imaging unit 5 may be a 2D optical camera (e.g. an RGB video camera, a webcam, or a still image camera that regularly acquires images), but may alternatively be an infrared camera (e.g. to work in darkness as well) or a 3D (depth) camera, such as a time-of-flight camera to provide distance information as e.g. used for monitoring the patient's activity as required in applications related to delirium detection or an ultrasound imaging device. The desired state is that the patient 2 on the bed 3 is fully within the field of view of the imaging unit 5.

In case a 3D (depth) camera is used as imaging unit (or if a 2D camera and a depth measurement unit (not shown in Fig. 1), e.g. a radar unit, are used) depth information is available within or along with the image data. From this depth information (and the image data) it is possible to detect information about the spatial relationship in 3D, in particular to detect occlusions and occluding objects.

Generally, a projection unit and temporal illumination of the field of view (during the image acquisition) in order to obtain information for detecting occlusions and occluding objects may be provided, but may be omitted in other embodiments, e.g. in an embodiment in which the imaging unit is a 3D camera.

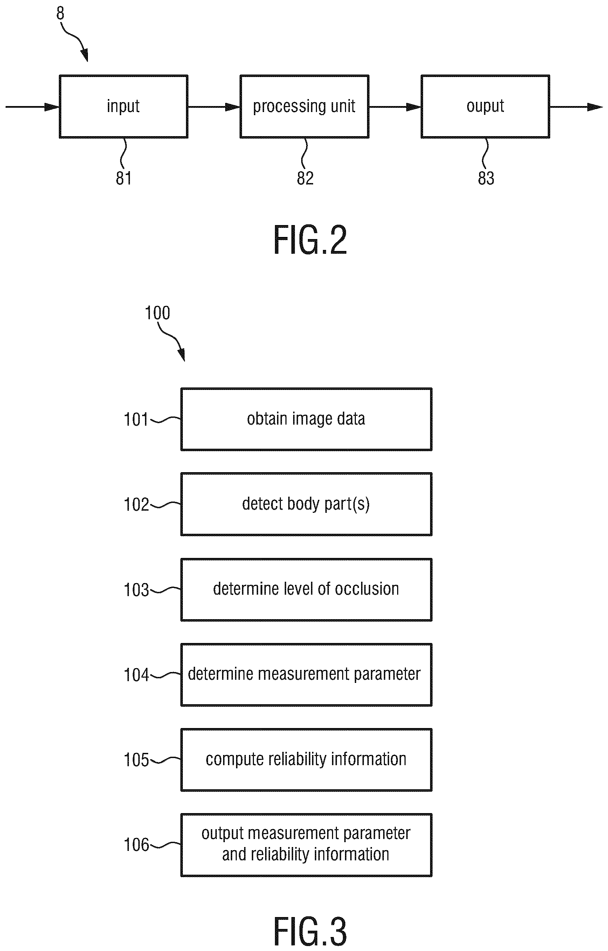

Fig. 2 shows a schematic diagram of an embodiment of a device 8 according to the present invention. The device 8 according to the present invention processes the image data acquired by the imaging unit 5 and computes a desired measurement parameter, which may e.g. be one or more of respiration rate, heart rate, Sp02, bed fall likelihood, bed exit detection, pressure ulcers, delirious movements, pain, facial expressions, subject motion, subject activity and blood pressure.

As shown in Fig. 2 the device 8 comprises an input interface 81 configured to obtain (i.e. receive or retrieve) image data acquired over time by an imaging unit from a field of view containing at least part of a subject, a processing unit 82, and an output interface 83 configured to output the measurement parameter and the computed reliability information. The input interface 81 and the output interface 83 may be separate interfaces or a common interface.

The input interface 81 may be directly coupled or connected to the imaging unit 5 or may obtain the image data from a storage, buffer, network, or bus, etc. The input may thus e.g. be (wired or wireless) communication interfaces or data interfaces, such as a Bluetooth interface, WiFi interface, LAN interface, HDMI interface, direct cable connect, or any other suitable interface allowing signal transfer to the device.

The processing unit 82 may be any kind of means configured to process the image data and determine the measurement parameter and the reliability information there from. It may be implemented in software and/or hardware, e.g. as a programmed processor or computer or app on a user device such as a smartphone, smartwatch, tablet, laptop, PC, workstation, etc.

The output interface 83 may generally be any means that outputs the determined measurement parameter and reliability information in visual or audible form, e.g. in text form, as image or diagram, as sound or spoken words, etc. For instance, the output interface 83 may be a display, a loudspeaker, a touchscreen, a computer monitor, the screen of a smartphone or tablet, etc. It may, additionally or alternatively, be configured to transmit the computed information to another device or to provide it for retrieval by another device (e.g. a smartphone, computer, tablet, etc.). It may thus generally be any (wired or wireless) communication interface or data interface or user interface.

Fig. 3 shows a schematic diagram of a first embodiment of a method 100 for monitoring a subject according to the present invention. The steps of the method may be carried out by the device 8, wherein the main steps of the method are carried out by the processing unit 82. The method 100 may e.g. be implemented as computer program running on a computer or processor.

In a first step 101 the image data are obtained. In a second step 102, within the obtained image data, one or more body parts of the subject, whose image data allow determination of a desired measurement parameter, are detected. In a third step 103 a level of occlusion of the detected one or more body parts is determined. In a fourth step 104 the desired measurement parameter is determined from the image data of at least one of the detected one or more body parts. In a fifth step 105 reliability information of the determined measurement parameter is computed based on the detected level of occlusion of the at least one of the detected one or more body parts whose image data have been used to determine the measurement parameter.

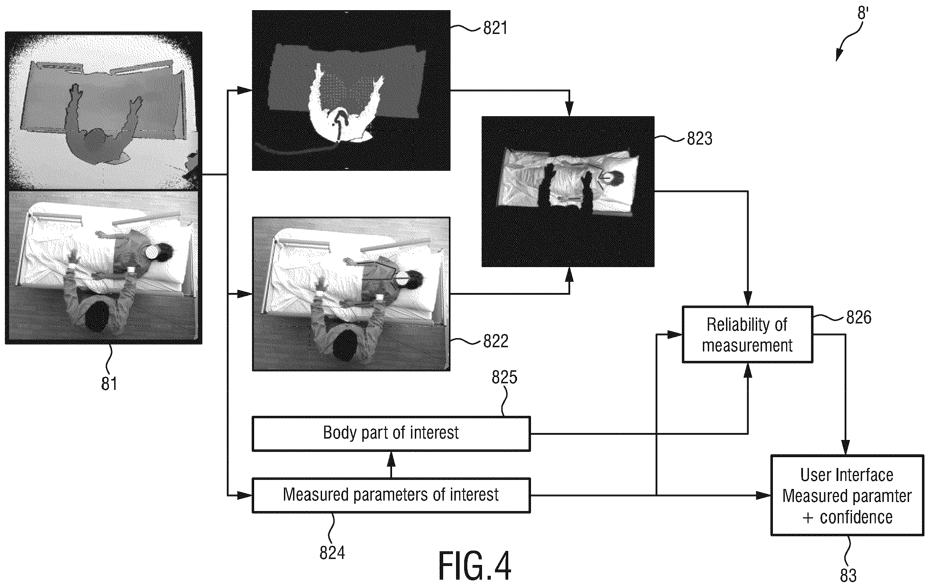

Fig. 4 shows another embodiment of a device 8' according to the present invention.

In this embodiment the imaging unit 5 may be a 3D IR camera that is located such that it can continuously monitor the scene of interest with the patient in view. The camera can be attached to the ceiling above the patient bed with a wide angle to have the complete patient in view. Depth data and IR image data are thus provided to the input interface 81.

The processor 82 may comprise a number of building blocks.

In a patient area and occlusion detection (PAOD) unit 821 the area where the patient is located is detected. In the case of the ICU this is the bed area, but in case of mobilization this can also be a chair next to the bed. Next to the patient area, the location of objects / persons obstructing the view of the patient area are being detected.

A known method as e.g. disclosed in WO 2020/127014 A1 may be used to detect the bed region as patient area of interest. In the ICU, most patients are the majority of the time in the bed. Further, it is preferred to detect possible objects/persons obscuring the view on the patient. The patient area detection (PAD) method may e.g. discard objects that are not the bed or patient, removing all objects of non-interest in the patient area of interest.

In an exemplary scenario the exact shape of the bed/patient area without occlusion by objects/persons is determined in a calibration procedure where there are no objects/persons overlapping the area. This information can be used to compare with the detected patient area (PA) at another moment in time. The area not being detected by the PA is occluded area.

In a more sophisticated embodiment the level of PA obscurity is used as a weighting factor of how well the PA can be observed. In this embodiment occluding objects are not either being discarded or kept by defining a binary mask (i.e. PA occluded vs. PA not-occluded), but weighted according to their type of occlusion. For instance, TV-screens or persons within the PA area that completely obscure the PA view would have a very high obscurity score. Bed sheets do not obstruct but obscure the view of the region interest (e.g. the legs). This may e.g. be characterized by a medium obscurity score. Unobstructed or obscured objects may have an unequivocally low obscurity score. This obscurity score can subsequently be used as a weighting factor for calculating the overall reliability score.

Another option is to use the information of the whole image and track motion of the persons/objects in the room and to the bed area. By using the motion history and understand the shape the occlusion can be determined.

In a patient body part/region recognition (PBPR) unit 822 the different body parts of the patient in the patient view are detected. In particular, the position of the patient body region/parts is identified. Knowing the location of the patient body parts is useful to understand which body part/region is occluded or obscured. A coarse method of detecting body regions can be done based on depth profiles. In this way, the body is divided into different regions, such as head, trunk, arms, and legs. With this division a reliability score can be defined for each region separately. The trunk may be useful for e.g. detection of breathing rate. The head may be important for facial expression detection. The arms may be useful for typical delirious hand movements.

In another embodiment the body position may be used as a whole, where the position of the body relative to the camera informs on how well the body part of interest can be monitored. For instance, patients lying on their belly will impede respiratory rate measurements as the trunk cannot be observed. In case of a top-view camera, patients in seated position may compromise facial expression recognition. Simple heuristics or body position recognition algorithms may be used to identify the total body position and can serve as an additional weighting factor for calculating the overall measurement reliability score.

A more detailed picture on the body part location can be derived using deep learning methods such as OpenPose.

With this method different joints can be identified. For instance, next to the arms, a more precise description of the hands can be obtained. This can be of interest for monitoring delirious behavior. If the output of the learning method gives a very low confidence value for a certain joint/body part, or skeleton, this indicates a possible occlusion. This information can be combined with the occlusion information from the previous step to derive information on the amount of occluded body parts.

For implementation efficiency, the above-mentioned two steps (patient and occlusion detection, body part/region recognition) may only be carried out when there is a change in the scene. The scene change can be detected by applying an efficient motion detection algorithm such as frame differencing.

In a statistics calculation unit 823 the outputs of the PAOD unit 821 and the PBPR unit 822 are combined to calculate statistics on the level of occlusion of the different patient body parts. Different statistics may be determined about the occluded body parts/region. Statistics of interest may include one or more of:

- Identification of body parts/regions covered
- Number of body parts/regions covered
- Percentage of the coverage of the different body parts/regions
- Period of time that the body part/region is covered
- Frequency of coverage
- Change in overall patient body position

In addition, statistics of the occluding people/objects can be also of interest. Occluding objects, such as monitors, trays, etc., tend to be static for a longer time. This information can be used to warn the staff members.

In a measurement unit 824 an algorithm to detect a desired measurement parameter is applied. In particular, video-based algorithms may be used to detect the one or more measurements of interest. Examples of possible measurements are respiration rate, pressure ulcers, bed exit detection, delirious movements, pain, etc. Based on the input image the algorithm is deriving the parameters. For detecting vital signal, such as heart rate, Sp02, etc. the known technique of remote photo-plethysmography may be applied, as e.g. described in Verkruysse et al., "Remote plethysmographic imaging using ambient light", Optics Express, 16(26), 22 December 2008, pp. 21434-21445. Respiration rate can e.g. be measured by detecting movements of the chest. Other measurements can be made by detecting characteristic movements of the subject.

In a body part of interest (BPI) computation unit 825 predefined body parts of interest are identified based on the measurement parameter of interest. If the measurement is breathing rate a good view on the trunk is needed. If there are several body parts that are of interest these can be prioritized. For each measurement parameter one or more preferred body parts/regions may be defined. This depends on the type of measurement under consideration, e.g. for respiratory rate the trunk may be used, for pressure ulcers the complete body may be used, for delirious movements the hands and the legs may be used.

- In a confidence of measurement computation unit 826, based on information provided by units 823, 824 and 825 measurement reliability, e.g. in the form of a reliability score or level, is calculated. Thus, the information of the measurement, joint/region of interest and statistics of occluded body parts/regions are combined. This information may be used in a model to define how reliable the obtained measurement is. If the body part/region of interest is occluded completely the measurement can be discarded and not be trusted and will have a score of 0 while when it is partially visible a score between 0-100 can indicate that the score is not fully reliable but should be taken with some caution. The confidence score can be obtained by making use of logic or more sophisticated machine learning techniques. Next to the proportion of occlusion the time of occlusion can be taken into account for the score as well. For prolonged occlusion the confidence can be lowered.

The user interface 83 may show (e.g. on a display screen) the measurement and the reliability, e.g. in the form of the reliability score. This may be done with color-coding or numerically. It can also give insight in coverage of the body part of interest so it can be adjusted in case of a static object.

To detect objects of interest, such as the bed region or a chair as patient area of interest, different methods may be used. According to WO 2020/127014 A1 multiple depth images of a scene are used to compute a noise variance map by computing pixel noise variances to determine object boundaries of one or more objects in the depth images, a depth confidence map by filtering depth values based on their distance to the depth camera, and a motion confidence map by filtering out pixel noise variances caused by motion of a person in the scene. Further, from the noise variance map, the depth confidence map and the motion confidence map, one or more candidate regions and their confidence in the depth images are computed, and the one or more candidate regions having the highest confidence are selected as final region of interest representing the object to be recognized, e.g. the bed region or bed border region. In this way the object can be automatically detected. A depth image of a scene, e.g. from a depth camera (time of flight camera), is used to detect variations at object boundaries (e.g. bed borders) and/or at occluding object boundaries (e.g. nurses) to remove objects. Further, combining a noise map with a depth map and a motion confidence map provides for effective contour detection and selecting the best final region. Still further, the object may be segmented and/or localized by use of the present invention. Further details of the detection can be found in WO 2020/127014 A1, which is herein incorporated by reference.

Another option uses 3D point clouds in world coordinates and applies corresponding decision logics. In a first step the input depth image is transformed into a 3D point cloud representation, based on known camera parameters. Working in the 3D point cloud structure offers many benefits such as being able to use world coordinate system and derive real measurements on the scene objects. After the transformation, the depth image has a point cloud form. In a second step the camera-viewing angle is corrected, such that the ground plane is always horizontal on the X-Y plane. To this end, it is assumed that the original 3D point clouds up to 0.5 meters above the furthest points constitute mostly ground pixels. These selected pixels are used to fit a 3D ground plane with a robust technique RANSAC (RANdom Sample Consensus). The RANSAC based estimation gives very robust solution due to its ability to handle outliers. Based on the estimated plane parameters, the 3D point cloud is rotated such that the ground plane is on X-Y plane. The estimation of the transformation parameters needs to be done only once unless the camera is re-positioned. In a third step, after the scene rectification, the Z-value of the point clouds represents the true measurements. The ground plane pixels (close to 0 meter) are then removed from the point cloud, resulting pixels corresponding to persons (patients/other persons) and other objects in the room. By examining the histogram of the Z-values of the remaining pixels, the peaking mode is extracted that lies within the range of 0.8 meters and 1.2 meters, which most likely corresponds to the patient bed height. Based on the estimated bed height, an asymmetrical normal distribution is used to generate the depth prior of all pixels in the 3D point cloud. The area above the reference bed height is assigned to a higher probability than the area below the reference bed height.

In a fourth step a point cloud segmentation is performed on the scene pixels (without ground plane). A seed pixel is chosen from the reference height (on the detected bed plane) which grows into an object (segment) based on a pre-defined connectivity measure. The measure is defined as a combination of 3D distance between a candidate pixel B and the reference pixel A, and their associated depth priors.

$$Conn(A,B) = EucDistance(A,B) - w * Max(DepthPrior(A), DepthPrior(B))$$

In the above, w is a weighting factor, *EucDistance*(A, B) determines the Euclidean distance between point A and B, and the maximum of the depth priors of the two points is used to make sure that only points with small distances to the reference point A and with high depth priors will be included in the region growing. Alternative measures can be used as well. For example, the Max operation can be replaced by mean/average. The idea is to incorporate both space connectivity and depth priors that are specific to the application. If not all pixels from the reference bed plane are scanned, then new seed pixels are selected to grow into new object segments. This could potentially lead to multiple object segments. Therefore in the next step some post-processing can be used to determine which object segments should be combined to form a relevant ROI.

In a fifth step, by applying the point cloud segmentation on the point cloud, a few object segments are selected as candidate clusters. To determine whether they should all belong to the same patient activity area connectivity between each pair of object segments is checked. An example implementation is to check if the two segments are separated by ground area. If not, the segments are mostly likely separated by occluding objects such as devices and body parts of other persons.

After the occlusion analysis, further post-processing such as cleaning-up of images by median filtering may be applied. Finally, the patient activity area is concluded. The resulting ROI point cloud and its associated 2D representation provide valuable inputs for subsequent analysis such as characterization of patient motion and patient pose estimation. For example, the body parts of the patients can be extracted from the ROI point cloud in a much easier way than from the full scene in the room.

An exemplary method of detecting body regions based on depth profiles as follows. In a first step patient area detection is performed. The patient area detection finds the area where the patient and relevant activities take place. It filters out clutters in the room and other persons/objects in the scene. The patient area detection also rectifies the scene, such that potential camera tilt is corrected and the ground plane is at Z = 0 plane. As an alternative, from the depth image a reasonable estimate of the bed location can be derived from the X/Y depth profiles.

In a second step patient presence detection is performed. For the presence detection, the height values on the top part of the bed are scanned. For example, the vertical scan lines of the height map will contain a significant peak when the patient is present in bed. In addition, the horizontal scan lines of the height map can be used to complement the information from the vertical scan lines. The two cases can be differentiated by applying a simple peak detector or using a light-weight classifier that makes use of the profile. Another option is to identify an empty bed profile in a calibration phase when installing the camera system. Or the system contains a standard database with standard bed profiles from different viewing angles. There can be multiple bed profiles to cover different possible bed angles like tilted with 30 degrees, no tilt, etc.

In a third step patient orientation detection is performed. From the height map of the bed area, the orientation of the bed can be derived. It can be assumed that the side with larger height values corresponds to the bed head. From this, the lying orientation of the patient is known. Alternatively, fiducial markers at the bed head that are included in the patient area can identify the top of the bed, or at least where the patients' head resides.

In a fourth step posture analysis can be performed. The changes of the poses can be tracked over time. As compared to a reference frame, when the patient changes his/her position from lying on the back to lying on the side, the depth profile shows a significant increase in height. By capturing this feature, the posture change of the patient can be tracked and corresponding statistics can be derived. These statistics could lead to actionable tasks or items that need follow-up. For instance, it can be kept track of time spent in a certain position and it can be determined whether repositioning is recommended to avoid possible pressure ulcers. In another embodiment posture change analysis can be done by applying OpenPose on detected body parts.

The present invention can be used e.g. in hospital wards where patients/persons are being monitored by means of a camera or other care facilities with camera-based subject monitoring.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

**1.** Device for monitoring a subject, the device comprising:

an input interface (81) configured to obtain image data acquired over time by an imaging unit from a field of view containing at least part of a subject;
a processing unit (82) configured to

- detect, within the obtained image data, one or more body parts of the subject, whose image data allow determination of a desired measurement parameter,
- determine a level of occlusion of the detected one or more body parts,
- determine the desired measurement parameter from the image data of at least one of the detected one or more body parts, and
- compute reliability information of the determined measurement parameter based on the detected level of occlusion of the at least one of the detected one or more body parts whose image data have been used to determine the measurement parameter; and

an output interface (83) configured to output the measurement parameter and the computed reliability information.

**2.** Device as claimed in claim 1,
wherein the processing unit (82) is configured to select the at least one of the detected one or more body parts whose image data shall be used to determine the measurement parameter based on the level of occlusion of the one or more body parts.

**3.** Device as claimed in any one of the preceding claims,
wherein the processing unit (82) is configured to detect a subject area in which the subject is located, in particular an area in which a bed and/or chair is located, and to detect the one or more body parts of the subject within the detected subject area.

4. Device as claimed in any one of claim 3,
wherein the processing unit (82) is configured to detect occluding objects and/or persons occluding at least part of the subject, bed and/or chair and to detect the subject area and/or the one or more body parts of the subject using the detected occluding objects and/or persons.

5. Device as claimed in claim 4,
wherein the processing unit (82) is configured to determine, for the detected occluding objects and/or persons, a level of obscurity indicating to which extent a respective occluding object and/or person occludes or obstructs the subject area, and to use the determined level of obscurity in the computation of the reliability information of the determined measurement parameter.

6. Device as claimed in any one of claim 3, 4 or 5,
wherein the processing unit (82) is configured to detect occluding objects and/or persons occluding at least part of the subject, bed and/or chair by tracking motion of objects and/or persons within the image data and determine if a tracked object and/or person is or will be an occluding object or person based on the tracked motion.

7. Device as claimed in any one of the preceding claims,
wherein the processing unit (82) is configured to

   - detect position and/or orientation of the subject,
   - determine, based on the detected position and/or orientation, a weighting factor indicating how well the one or more body parts of the subject can be detected and/or how well the desired measurement parameter can be determined from the image data of at least one of the detected one or more body parts, and
   - use the determined weighting factor in the computation of the reliability information of the determined measurement parameter.

8. Device as claimed in any one of the preceding claims,
wherein the processing unit (82) is configured to detect scene changes, in particular body motions and/or position changes of the subject and/or changes in occlusion of one or more body parts of the subject, and to carry out body part detection and occlusion detection only when a scene change happens.

9. Device as claimed in any one of the preceding claims,
wherein the processing unit (82) is configured to determine the level of occlusion of the detected one or more body parts including one or more of:

   - identification of occluded body parts,
   - number of occluded body parts,
   - percentage of the occlusion of the respective body parts,
   - duration of occlusion of the respective body parts,
   - frequency of occlusion,
   - change in overall body position of the subject, and
   - type of occluding object and/or person.

10. Device as claimed in any one of the preceding claims,
wherein the processing unit (82) is configured to determine, as the desired measurement parameter, one or more of respiration rate, heart rate, Sp02, bed fall likelihood, bed exit detection, pressure ulcers, delirious movements, pain, facial expressions, subject motion, subject activity and blood pressure.

11. Device as claimed in any one of the preceding claims,
wherein the processing unit (82) is configured to compute the reliability information of the determined measurement parameter based on the detected level of occlusion by reducing the reliability with increasing level of occlusion.

12. Device as claimed in claim 11,
wherein the processing unit (82) is configured to compute the reliability information of the determined measurement parameter based on the duration of occlusion in addition to the detected level of occlusion by reducing the reliability with increasing duration of occlusion.

**13.** System for monitoring a subject, the system comprising:

- an imaging unit (5) configured to acquire image data over time from a field of view (6) containing at least part of a subject, and
- a device (8) defined in any one of the preceding claims for monitoring a subject based on the acquired image data.

**14.** Method for monitoring a subject, the method comprising:

- obtaining image data acquired over time by an imaging unit from a field of view containing at least part of a subject,
- detecting, within the obtained image data, one or more body parts of the subject, whose image data allow determination of a desired measurement parameter,
- determining a level of occlusion of the detected one or more body parts,
- determining the desired measurement parameter from the image data of at least one of the detected one or more body parts,
- computing reliability information of the determined measurement parameter based on the detected level of occlusion of the at least one of the detected one or more body parts whose image data have been used to determine the measurement parameter, and
- outputting the measurement parameter and the computed reliability information.

**15.** Computer program comprising program code means for causing a computer to carry out the steps of the method as claimed in claim 14 when said computer program is carried out on the computer.

FIG.1

8

| input | | processing unit | | ouput |

81       82       83

# FIG.2

100

| 101 | obtain image data |
| 102 | detect body part(s) |
| 103 | determine level of occlusion |
| 104 | determine measurement parameter |
| 105 | compute reliability information |
| 106 | output measurement parameter and reliability information |

# FIG.3

FIG.4

Europäisches
Patentamt

European
Patent Office

Office européen
des brevets

# EUROPEAN SEARCH REPORT

Application Number

EP 21 20 6924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 643 235 A1 (KONINKLIJKE PHILIPS NV [NL]) 29 April 2020 (2020-04-29) * paragraphs [0014], [0028] – [0041]; claim 1; figure 3 * | 1-15 | INV. A61B5/11 A61B5/00 A61G7/018 G16H40/00 |
| X | US 2019/209046 A1 (ADDISON PAUL S [GB] ET AL) 11 July 2019 (2019-07-11) * paragraphs [0081] – [0088], [0098] – [0109], [0118], [0154] * | 1-15 | |
| Y | US 2016/125620 A1 (HEINRICH ADRIENNE [NL] ET AL) 5 May 2016 (2016-05-05) * paragraphs [0056] – [0058], [0073], [0087] – [0092] * | 1-15 | |
| Y | US 2018/014790 A1 (FALCK THOMAS [DE] ET AL) 18 January 2018 (2018-01-18) * paragraph [0065] * | 1-15 | |

TECHNICAL FIELDS SEARCHED (IPC)

A61B
A61G
G16H

The present search report has been drawn up for all claims

| Place of search | Date of completion of the search | Examiner |
|---|---|---|
| Berlin | 13 April 2022 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 6924

This annex lists the patent family members relating to the patent documents cited in the above-mentioned European search report.
The members are as contained in the European Patent Office EDP file on
The European Patent Office is in no way liable for these particulars which are merely given for the purpose of information.

13-04-2022

| Patent document cited in search report | | Publication date | Patent family member(s) | | Publication date |
|---|---|---|---|---|---|
| EP 3643235 | A1 | 29-04-2020 | CN | 112912001 A | 04-06-2021 |
| | | | EP | 3643235 A1 | 29-04-2020 |
| | | | EP | 3870045 A1 | 01-09-2021 |
| | | | JP | 2022514167 A | 10-02-2022 |
| | | | US | 2021358616 A1 | 18-11-2021 |
| | | | WO | 2020083772 A1 | 30-04-2020 |
| US 2019209046 | A1 | 11-07-2019 | AU | 2018400475 A1 | 16-07-2020 |
| | | | CA | 3086527 A1 | 11-07-2019 |
| | | | CN | 111565638 A | 21-08-2020 |
| | | | EP | 3713486 A1 | 30-09-2020 |
| | | | US | 2019209046 A1 | 11-07-2019 |
| | | | WO | 2019135877 A1 | 11-07-2019 |
| US 2016125620 | A1 | 05-05-2016 | CN | 107072548 A | 18-08-2017 |
| | | | EP | 3214996 A1 | 13-09-2017 |
| | | | JP | 6588978 B2 | 09-10-2019 |
| | | | JP | 2017536880 A | 14-12-2017 |
| | | | US | 2016125620 A1 | 05-05-2016 |
| | | | WO | 2016071314 A1 | 12-05-2016 |
| US 2018014790 | A1 | 18-01-2018 | CN | 107257651 A | 17-10-2017 |
| | | | EP | 3262544 A1 | 03-01-2018 |
| | | | JP | 2018509962 A | 12-04-2018 |
| | | | US | 2018014790 A1 | 18-01-2018 |
| | | | WO | 2016135069 A1 | 01-09-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020127014 A1

**Non-patent literature cited in the description**

- **VERKRUYSSE et al.** Remote plethysmographic imaging using ambient light. *Optics Express,* 22 December 2008, vol. 16 (26), 21434-21445